# EUROPEAN PATENT APPLICATION

(11) **EP 4 748 823 A1**
(43) Date of publication of application: **27.05.2026**
(21) Application number: 25216311.8
(22) Date of filing: 17.11.2025
(51) Int. Cl.: C07C 51/47, C07C 51/48, C07C 55/10

(54) **BIO-BASED SUCCINIC ACID COMPOSITION, PREPARATION METHOD THEREFOR, AND POLYESTER PREPARED THEREFROM**

(30) Priority: 22.11.2024 CN 202411676804
(71) Applicant: Shanghai Kingfa Science & Technology Co., Ltd, Shanghai 201714 (CN); Liaoning Kingfa Biomaterial Co., Ltd., Panjin City, Liaoning Province 124000 (CN); Zhuhai Kingfa Biomaterial Co., Ltd., Zhuhai, Guangdong 519050 (CN)
(72) Inventor: Lu, Changli, Shanghai, 201714 (CN); Chen, Pingxu, Shanghai, 201714 (CN); Ye, Nanbiao, Shanghai, 201714 (CN); Zeng, Xiangbin, Shanghai, 201714 (CN); Fu, Xuejun, Shanghai, 201714 (CN); Guo, Zhilong, Shanghai, 201714 (CN); Wang, Chaojun, Shanghai, 201714 (CN); Ouyang, Chunping, Shanghai, 201714 (CN); Zhang, Erjie, Shanghai, 201714 (CN); Yao, Yi, Shanghai, 201714 (CN)
(74) Representative: Petraz, Gilberto Luigi

(57) **Abstract**

The present invention provides a bio-based succinic acid composition, a preparation method therefor, and a polyester prepared therefrom. The bio-based succinic acid composition includes bio-based succinic acid and a metal ion; and a mass content of the metal ion in the bio-based succinic acid composition is 10-350 ppm. In the present invention, by adopting the bio-based succinic acid composition containing the metal ion as a raw material for the polyester and controlling the content of the metal ion in the bio-based succinic acid composition within a specific range, the obtained polyester has better mechanical properties and heat retention stability.

## Description

### TECHNICAL FIELD

The present invention belongs to the technical field of preparation of polyesters, and particularly relates to a bio-based succinic acid composition, a preparation method therefor, and a polyester prepared therefrom.

### BACKGROUND

Succinic acid (also known as butanedioic acid), as a C4 compound, has been widely used in industries, such as medicine, pesticides, dyes, spices, paints, foods, and plastics, and can also be used for synthesizing organic chemicals, such as 1,4-butanediol, tetrahydrofuran, and γ-butyrolactone, and biodegradable materials, such as poly(butylene succinate) (PBS), thus having a good biological application prospect. Compared with the production of succinic acid with petroleum resources as raw materials, the production of succinic acid with biomass resources as raw materials has advantages, such as renewable raw materials, low cost, little pollution, and an ability to address environmental problems (such as the greenhouse effect), and has become a research hotspot in recent years.

A fermentation method is mostly adopted for the production of succinic acid with the biomass resources as the raw materials. For example, the succinic acid is synthesized by adopting a microbial conversion manner or a combination manner of microbial conversion and chemical conversion. During the microbial conversion, a decrease in pH will lead to reduced metabolic activity of microorganisms, or even cessation of activity, thereby resulting in deterioration of a manufacturing yield. Therefore, a neutralizing agent is usually used to regulate the pH of a fermentation system. Then, a neutralized succinic acid fermentation solution is purified to obtain a succinic acid product with a purity of greater than 99%. However, the obtained high-purity succinic acid product may still contain nitrogen elements contained in the biomass resources, nitrogen elements derived from microorganisms or enzymes, nitrogen elements such as ammonia used in a purification process, sulfur elements, inorganic acids, organic acids, metal cations, etc. In addition, the aforementioned neutralizing agent includes ammonia, ammonium carbonate, urea, alkali (alkaline earth) metal hydroxides (such as NaOH, KOH, Ca(OH)₂, Mg(OH)₂, etc.), alkali (alkaline earth) metal carbonates (such as Na₂CO₃, K₂CO₃, CaCO₃, and MgCO₃), etc., which will further lead to residues of nitrogen elements and metal ions in the succinic acid.

In a case that the content of metal cations in the succinic acid exceeds 600 ppm, the reactivity of succinic acid is weakened, and the product quality of a resulting polyester is deteriorated, resulting in poor thermal stability of the polyester. In particular, in a case of long-term heat retention, the polyester will undergo thermal decomposition in processing equipment, such as an extruder or an injection molding machine, resulting in a rapid decline in the performance of a molded product.

Therefore, the development of a bio-based succinic acid composition capable of improving the thermal stability of a polyester to enable the polyester to still have a high retention rate of tensile strength under long-term heat retention is an urgent problem to be solved in the art.

### SUMMARY

In view of the shortcomings of the prior art, the purpose of the present invention is to provide a bio-based succinic acid composition, a preparation method therefor, and a polyester prepared therefrom. The present invention is used to solve the problem of poor heat retention stability of polyesters prepared from bio-based succinic acid as a raw material in the prior art.

To achieve the purpose, the present invention adopts the following technical solutions.

In a first aspect, the present invention provides a bio-based succinic acid composition, where the bio-based succinic acid composition includes bio-based succinic acid and a metal ion; and a mass content of the metal ion in the bio-based succinic acid composition is 10-350 ppm.

In the present invention, by using the bio-based succinic acid containing the metal ion as a raw material and controlling the content of the metal ion in the bio-based succinic acid composition within a specific range, the polyester prepared and obtained under same reaction conditions has superior mechanical properties and heat retention stability and has a low melt index.

In the present invention, the mass content of the metal ion in the bio-based succinic acid composition is 10-350 ppm, and for example, may be 10 ppm, 20 ppm, 30 ppm, 40 ppm, 50 ppm, 60 ppm, 70 ppm, 80 ppm, 90 ppm, 100 ppm, 110 ppm, 120 ppm, 130 ppm, 140 ppm, 150 ppm, 160 ppm, 170 ppm, 180 ppm, 190 ppm, 200 ppm, 210 ppm, 220 ppm, 230 ppm, 240 ppm, 250 ppm, 260 ppm, 270 ppm, 280 ppm, 290 ppm, 300 ppm, 310 ppm, 320 ppm, 330 ppm, 340 ppm, 350 ppm, or a range between any of the above numerical values.

Preferably, the mass content of the metal ion in the bio-based succinic acid composition is 20-280 ppm, more preferably 60-200 ppm.

In the present invention, when the mass content of the metal ion is within the above range, the obtained polyester has better mechanical properties and heat retention stability. When the content of the metal ion is too high, the reactivity of succinic acid is affected, thereby affecting the performance of the polyester. In addition, obtaining bio-based succinic acid with a low metal ion content is a goal pursued by those skilled in the art. In order to achieve an extremely low (< 10 ppm) metal ion content, purification several times and inputting of expensive purification equipment are required, which is economically disadvantageous. However, the bio-based succinic acid composition provided by the present invention will not increase the cost obviously, and can obtain a high-performance polyester material.

In the present invention, "bio-based" in the bio-based succinic acid composition means that a main component of the succinic acid composition is derived from a biomass resource.

Preferably, the metal ion includes any one or a combination of at least two of Na⁺, K⁺, Mg²⁺, and Ca²⁺.

In the present invention, the metal ion may be a residual metal ion derived from the process of preparing the bio-based succinic acid using the biomass resource as a raw material, and may also be derived from a compound containing a metal ion that is additionally added as needed.

In the present invention, when the metal ion is the residual metal ion derived from the process of preparing the bio-based succinic acid using the biomass resource as the raw material, the content of the metal ion in the succinic acid may be reduced by post-treatment, so that the final content of the metal ion in the bio-based succinic acid composition is within a specific range; and a post-treatment method includes an extraction and an adsorption method using a cation exchange resin column.

Preferably, the metal ion includes the Na⁺, the K⁺, the Mg²⁺, and the Ca²⁺.

Preferably, a mass content of the Na⁺ or K⁺ in the bio-based succinic acid composition is ≤ 60 ppm, and for example, may be 5 ppm, 10 ppm, 15 ppm, 20 ppm, 25 ppm, 30 ppm, 35 ppm, 40 ppm, 45 ppm, 50 ppm, 55 ppm, 60 ppm, or a range between any of the above numerical values; and the mass content is further preferably 2-50 ppm, more preferably 5-30 ppm.

Preferably, a mass content of the Mg²⁺ in the bio-based succinic acid composition is ≤ 50 ppm, and for example, may be 5 ppm, 10 ppm, 15 ppm, 20 ppm, 25 ppm, 30 ppm, 35 ppm, 40 ppm, 45 ppm, 50 ppm, or a range between any of the above numerical values; and the mass content is further preferably 5-40 ppm, more preferably 10-30 ppm.

Preferably, a mass content of the Ca²⁺ in the bio-based succinic acid composition is ≤ 220 ppm, and for example, may be 10 ppm, 20 ppm, 30 ppm, 40 ppm, 50 ppm, 60 ppm, 70 ppm, 80 ppm, 90 ppm, 100 ppm, 110 ppm, 120 ppm, 130 ppm, 140 ppm, 150 ppm, 160 ppm, 170 ppm, 180 ppm, 190 ppm, 200 ppm, 210 ppm, 220 ppm, or a range between any of the above numerical values; and the mass content is further preferably 20-170 ppm, more preferably 70-130 ppm.

Preferably, under conditions of a temperature of 25°C and a concentration of 0.1 mol/L, a pH value of an aqueous solution of the bio-based succinic acid composition is ≤ 2.8, and for example, may be 1, 1.1, 1.2, 1.3, 1.4, 1.5, 1.6, 1.8, 2, 2.1, 2.2, 2.3, 2.4, 2.5, or a range between any of the above numerical values; and the pH value is more preferably ≤ 2.6, particularly preferably ≤ 2.4.

Under normal circumstances, under the conditions of 25°C and a concentration of 0.1 mol/L, the pH value of succinic acid is 2.7. When succinic acid reacts with butanediol to synthesize an aliphatic polyester, a lower pH value of the system is more conducive to enhancing the reactivity and improving the reaction efficiency and reaction degree, thereby shortening the polymerization residence time and obtaining the aliphatic polyester with superior quality indicators, such as an acid number and a color value, and better thermal stability. In the present invention, when a neutralizing agent is used to regulate the pH of a fermentation system, the pH value of the aqueous solution of the bio-based succinic acid composition may be reduced by regulating the addition amount of the neutralizing agent, and a polyester with better performance may be prepared and obtained by using the bio-based succinic acid composition with a low pH value as a raw material. Preferably, the pH value of the aqueous solution of the bio-based succinic acid composition is 2.6 or below.

In a second aspect, the present invention provides a method for preparing the bio-based succinic acid composition according to the first aspect, where the preparation method includes the following steps:
(1) using a biomass resource as a raw material to prepare and obtain a succinic acid stock solution; and
(2) purifying the succinic acid stock solution obtained in the step (1) to obtain the bio-based succinic acid composition, where a method for the purifying includes an extraction method and an adsorption method using a cation exchange resin column.

In the present invention, in the preparation method, the bio-based succinic acid composition with a high purity and a low metal ion content can be obtained through purification by extraction and the cation exchange resin column.

Preferably, a content of succinic acid in the succinic acid stock solution is 70-95 g/L, and for example, may be 70 g/L, 72 g/L, 75 g/L, 78 g/L, 80 g/L, 82 g/L, 85 g/L, 88 g/L, 90 g/L, 92 g/L, 95 g/L, or a range between any of the above numerical values.

Preferably, the extraction method includes forward cross-current extraction and reverse cross-current extraction.

Preferably, an extractant for the forward cross-current extraction includes a phosphate ester extractant.

Preferably, the phosphate ester extractant includes at least one of diisooctyl phosphate, diethyl phosphate, or triethyl phosphate.

In the present invention, based on a volume of 1 L of the succinic acid stock solution, a mass of the extractant for the forward cross-current extraction is 20-55 g, and for example, may be 20 g, 22 g, 24 g, 26 g, 28 g, 30 g, 32 g, 34 g, 36 g, 38 g, 40 g, 42 g, 44 g, 46 g, 48 g, 50 g, 52 g, 54 g, 55 g, or a range between any of the above numerical values, more preferably 25-45 g, and particularly preferably 30-40 g.

Preferably, an extractant for the reverse cross-current extraction is water.

In the present invention, based on a volume of 1 L of a solution containing succinic acid obtained by the forward cross-current extraction, a mass of the extractant for the reverse cross-current extraction is 30-95 g, and for example, may be 30 g, 32 g, 35 g, 38 g, 40 g, 42 g, 45 g, 48 g, 50 g, 52 g, 55 g, 58 g, 60 g, 62 g, 65 g, 68 g, 70 g, 72 g, 75 g, 78 g, 80 g, 82 g, 85 g, 88 g, 90 g, 92 g, 95 g, or a range between any of the above numerical values, more preferably 40-75 g, and particularly preferably 50-70 g.

In the present invention, a number of times of the forward cross-current extraction and the reverse cross-current extraction is each independently ≥ 1 time, and for example, may be 1 time, 2 times, 3 times, 4 times, 5 times, 6 times, 7 times, 8 times, or a range between any of the above numerical values.

Preferably, the cation exchange resin column is capable of adsorbing at least one of Na⁺, K⁺, Mg²⁺, and Ca²⁺

In the present invention, the cation exchange resin is a 732-type cation exchange resin; and the cation exchange resin includes, but is not limited to: Amberlite IR-120, Dowex-50; Germany: Lewatit-100; and Japan: Diaion SK-1.

Preferably, a column loading flow rate of the adsorption method using the cation exchange resin column is 0.5-2.8 BV/h, and for example, may be 0.5 BV/h, 0.6 BV/h, 0.8 BV/h, 1 BV/h, 1.1 BV/h, 1.2 BV/h, 1.3 BV/h, 1.4 BV/h, 1.5 BV/h, 1.6 BV/h, 1.7 BV/h, 1.8 BV/h, 1.9 BV/h, 2 BV/h, 2.1 BV/h, 2.2 BV/h, 2.3 BV/h, 2.4 BV/h, 2.5 BV/h, 2.6 BV/h, 2.7 BV/h, 2.8 BV/h, or a range between any of the above numerical values; and the column loading flow rate is further preferably 0.8-2 BV/h, more preferably 1-1.3 BV/h.

In the present invention, after passing through the cation exchange resin column, the adsorption method using the cation exchange resin column further includes a step of elution with water.

Preferably, a mass of the water is 2-7 times that of a solution to be eluted, and for example, may be 2 times, 3 times, 4 times, 5 times, 6 times, 7 times, or a range between any of the above numerical values.

In the present invention, the solution to be eluted refers to a solution containing succinic acid before passing through the cation exchange resin.

Preferably, the biomass resource includes a plant resource and/or an animal resource, preferably the plant resource.

In the present invention, the plant resource refers to a biomass resource capable of converting light energy of the sun into the form of starch or cellulose and storing the same through photosynthesis; the animal resource refers to a biomass resource that grows and develops by preying on plant bodies; and the plant animal or the animal resource further includes a product obtained by processing the plant animal or the animal resource.

In the present invention, exemplarily, the plant resource includes, but is not limited to, wood, rice straw, rice husk, rice bran, aged rice, corn, sugarcane, cassava, corn straw, cassava starch residue, bagasse, plant oil residue, buckwheat, soybean, food waste, and the like.

In the present invention, the preparation method further includes converting the biomass resource into a carbon source, and then using the carbon source as a raw material to prepare and obtain the succinic acid stock solution. A method for converting the biomass resource into the carbon source includes, but is not limited to, chemical treatment, physical treatment, biological treatment, and the like. Exemplarily, the chemical treatment may adopt acid treatment (for example, treatment with strong acids, such as sulfuric acid, nitric acid, hydrochloric acid, and phosphoric acid), alkali treatment, ammonia freezing, distillation and explosion treatment, solvent extraction treatment, supercritical fluid treatment, oxidizing agent treatment, and the like. The physical treatment may be crushing treatment, distillation and explosion treatment, microwave treatment, electron ray irradiation treatment, and the like. The biological treatment may be microbial treatment, enzymatic treatment, and the like.

In the present invention, the carbon source exemplarily includes, but is not limited to, hexoses, such as glucose, mannose, galactose, fructose, sorbitol, and tagatose; pentoses, such as arabinose, xylose, ribose, xylulose, and ribulose; and disaccharides or polysaccharides, such as pentosan, sucrose, starch, and cellulose; and the carbon source preferably includes the glucose, the fructose, and the xylose, and particularly preferably includes the glucose.

Preferably, a method for preparing and obtaining the succinic acid stock solution in the step (1) includes a microbial fermentation method and/or a chemical conversion method, preferably the microbial fermentation method.

In the present invention, a microorganism used in the microbial fermentation method is capable of producing a dicarboxylic acid, and exemplarily includes: anaerobic bacteria, facultative anaerobic bacteria, aerobic bacteria, and the like; the anaerobic bacteria may be, for example, of the genus *Anaerobiospirillum* (US5143833A); the facultative anaerobic bacteria may be, for example, of the genus *Actinobacillus* (US5504004A), the genus *Escherichia* (US5770435A), and the like; the aerobic bacteria may be, for example, of the genus *Corynebacterium* (JPH0 11-113588 communique or CN103183813A), and these references are incorporated herein by reference; and the aerobic bacteria, such as those of the genus *Corynebacterium,* are preferably used.

As *Corynebacterium* bacteria, microorganisms belonging to the genus *Corynebacterium,* microorganisms belonging to the genus *Brevibacterium,* or microorganisms belonging to the genus *Arthrobacter* may be enumerated, where preferred microorganisms may be enumerated as those belonging to the genus *Corynebacterium* or the genus *Brevibacterium,* and more preferred microorganisms may be enumerated as those belonging to *Corynebacterium glutamicum, Brevibacterium flavum, Brevibacterium ammoniagenes,* or *Brevibacterium lactofermentum.*

During microbial conversion, when the pH decreases, the metabolic activity of the microorganism is reduced or ceased, resulting in deterioration of a manufacturing yield or microorganism death. Therefore, a neutralizing agent is usually used to regulate the pH of a fermentation system. Usually, the pH of the reaction system is measured by a pH sensor, and then the pH is adjusted to a specified pH range by adding the neutralizing agent. A method for adding the neutralizing agent is particularly limited, which may be continuous addition or intermittent addition. The pH may be adjusted to a range that allows the most effective activity according to the type of the microorganism, such as bacteria or fungi, used; and the pH is usually 4-10, and preferably 6-9.

As the neutralizing agent, ammonia, ammonium carbonate, urea, alkali metal hydroxides, alkaline earth metal hydroxides, alkali metal carbonates, and alkaline earth metal carbonates may be enumerated; and the ammonia, the ammonium carbonate, and the urea are preferred. In addition, as the above alkali (alkaline earth) metal hydroxides, NaOH, KOH, Ca(OH)₂, Mg(OH)₂, or mixtures thereof may be enumerated; and as the alkali (alkaline earth) metal carbonates, Na₂CO₃, K₂CO₃, CaCO₃, MgCO₃, or mixtures thereof may be enumerated.

In the present invention, the succinic acid stock solution prepared and obtained by using the biomass resource as the raw material in the step (1) may be prepared by a conventional method in the art, and for example, may be prepared by referring to a method in CN118459331A.

Preferably, the method for the purifying further includes decolorization with activated carbon and/or filtration.

Preferably, in the decolorization with activated carbon, based on a volume of 1 L of a solution to be decolorized, a mass of the activated carbon is 0.5-10 g, and for example, may be 0.5 g, 1 g, 2 g, 3 g, 4 g, 5 g, 5.2 g, 5.5 g, 5.8 g, 6 g, 6.2 g, 6.5 g, 6.8 g, 7 g, 7.2 g, 7.5 g, 7.8 g, 8 g, 8.5 g, 9 g, 9.5 g, 10 g, or a range between any of the above numerical values.

In the present invention, the solution to be decolorized may be the succinic acid stock solution, and may also be a solution obtained after other purification treatments, for example, a solution obtained after at least one purification treatment, such as extraction, adsorption using a cation exchange resin column, and filtration, of the succinic acid stock solution.

Preferably, a temperature of the decolorization with activated carbon is 60-85°C, and a time is 30-65 min.

In the present invention, the filtration includes filtration using an ultrafiltration membrane; and a pore size of the ultrafiltration membrane is 20-80 nm, and for example, may be 20 nm, 25 nm, 30 nm, 35 nm, 40 nm, 45 nm, 50 nm, 55 nm, 60 nm, 65 nm, 70 nm, 75 nm, or 80 nm, or a range between any of the above numerical values.

Preferably, a temperature of the filtration is 40-60°C, and for example, may be 40°C, 42°C, 44°C, 46°C, 48°C, 50°C, 52°C, 54°C, 56°C, 58°C, or 60°C, or a range between any of the above numerical values.

Preferably, after the purifying, the preparation method further includes a step of reduced-pressure distillation and/or cooling crystallization.

In the present invention, a temperature of the reduced-pressure distillation is 65-75°C, a pressure is -0.07 - -0.1 MPa, and the distillation is stopped when the content of succinic acid is greater than 15 wt%; and the concentrated succinic acid solution is subjected to the cooling crystallization at a low temperature (≤ 8°C) to obtain the bio-based succinic acid composition.

As a preferred technical solution of the present invention, the preparation method includes the following steps:
(1) using the biomass resource as the raw material to prepare and obtain the succinic acid stock solution with a succinic acid content of 70-95 g/L;
(2) subjecting the succinic acid stock solution obtained in the step (1) to forward cross-current extraction with a phosphate ester extractant to obtain a succinic acid-loaded organic phase; and then, subjecting the succinic acid-loaded organic phase to reverse cross-current extraction with water to obtain an aqueous solution of succinic acid, where based on a volume of 1 L of the succinic acid stock solution, a mass of the phosphate ester extractant is 20-55 g; based on a volume of 1 L of the succinic acid-loaded organic phase, a mass of the water is 30-95 g; and a number of times of the forward cross-current extraction and the reverse cross-current extraction is each independently ≥ 1 time;
(3) allowing the aqueous solution of succinic acid obtained in the step (2) to pass through a cation exchange resin column at a flow rate of 0.5-2.8 BV/h, and performing elution with water with a mass 2-7 times that of the aqueous solution of succinic acid to obtain a succinic acid effluent, where the elution is performed with the water with a weight 2-7 times that of the aqueous solution of succinic acid;
(4) sequentially subjecting the succinic acid effluent obtained in the step (3) to decolorization with activated carbon and filtration to obtain a succinic acid filtrate, where based on a volume of 1 L of the succinic acid effluent, a mass of the activated carbon is 0.5-10 g; a temperature of the decolorization with activated carbon is 60-85°C, and a time is 30-65 min; and the filtration is performed using an ultrafiltration membrane, and a temperature of the filtration is 30-60°C; and
(5) subjecting the succinic acid filtrate obtained in the step (4) to reduced-pressure distillation and cooling crystallization to obtain the bio-based succinic acid composition.

In the present invention, the method for the purifying further includes a calcium salt method, an electrodialysis method, and the like.

In a third aspect, the present invention provides a polyester, where raw materials for preparing the polyester include the bio-based succinic acid composition described in the first aspect.

The polyester produced by using the specific bio-based succinic acid composition of the present invention as a raw material has a shorter residence time.

Preferably, the polyester includes the following components:
component A:
based on a total molar amount of 100 mol% of the component A, being a dicarboxylic acid compound including the following components:
   a1, being 65-100 mol% of bio-based succinic acid and/or a derivative of an ester thereof, and
   a2, being 0-35 mol% of adipic acid and/or a derivative of an ester thereof;
and component B: being 1,4-butanediol with a molar amount at least equal to that of the component A, where the bio-based succinic acid is the bio-based succinic acid composition described in the first aspect.

In the present invention, a molar ratio of the component B to the component A is (1-3):1, for example, may be 1:1, 1.2:1, 1.5:1, 1.8:1, 2:1, 2.2:1, 2.5:1, 2.8:1, 3:1, or a range between any of the above numerical values, more preferably (1-2):1.

In the present invention, the phrase "the polyester includes the following components" means that a molecular structure of the polyester includes a structural unit derived from the component A and a structural unit derived from the component B; and the phrase "a dicarboxylic acid compound including the following components" means that in the molecular structure of the polyester, a dicarboxylic acid structural unit part includes a structural unit derived from the component a1 and a structural unit derived from the component a2.

In the present invention, the 65-100 mol%, for example, may be 65 mol%, 66 mol%, 68 mol%, 70 mol%, 72 mol%, 74 mol%, 76 mol%, 78 mol%, 80 mol%, 82 mol%, 84 mol%, 86 mol%, 88 mol%, 90 mol%, 92 mol%, 94 mol%, 96 mol%, 98 mol%, 100 mol%, or a range between any of the above numerical values.

In the present invention, the 0-35 mol%, for example, may be 0 mol%, 2 mol%, 4 mol%, 6 mol%, 8 mol%, 10 mol%, 12 mol%, 14 mol%, 16 mol%, 18 mol%, 20 mol%, 22 mol%, 24 mol%, 26 mol%, 28 mol%, 30 mol%, 32 mol%, 34 mol%, 35 mol%, or a range between any of the above numerical values.

Preferably, based on the total molar amount of 100 mol% of the component A, the dicarboxylic acid compound includes the following components:
a1, 72-82 mol% of the bio-based succinic acid and/or a derivative of an ester thereof, and
a2, 18-28 mol% of the adipic acid and/or a derivative of an ester thereof.

In the present invention, a derivative of a succinic acid ester includes an alkyl succinate; exemplarily, the alkyl succinate may be at least one of dimethyl succinate, diethyl succinate, di-n-propyl succinate, diisopropyl succinate, di-n-butyl succinate, diisobutyl succinate, di-tert-butyl succinate, di-n-pentyl succinate, diisopentyl succinate, and di-n-hexyl succinate; and the alkyl succinate may be an alkyl ester formed from succinic acid or an alkyl ester formed from succinic anhydride, and dimethyl succinate formed from succinic anhydride is preferably used.

In the present invention, a derivative of an adipic acid ester includes an alkyl adipate; exemplarily, the alkyl adipate may be at least one of dimethyl adipate, diethyl adipate, di-n-propyl adipate, diisopropyl adipate, di-n-butyl adipate, diisobutyl adipate, di-tert-butyl adipate, di-n-pentyl adipate, diisopentyl adipate, and di-n-hexyl adipate; and the alkyl adipate may be an alkyl ester formed from adipic acid or an alkyl ester formed from adipic anhydride.

In the present invention, a dicarboxylic acid or a derivative of an ester thereof may be used separately or used in the form of a mixture of two or more.

In the present invention, according to the standard ISO 1133-2-2012 and under conditions of 190°C and 2.16 kg, a melt index of the polyester is ≤ 8.0 g/10 min, further preferably 3-7.0 g/10 min.

Preferably, an initial tensile strength of the polyester is ≥ 22 MPa, more preferably ≥ 37 MPa.

Preferably, after heat retention at 180°C for 10 min, a retention rate of tensile strength of the polyester is > 70%, more preferably > 82%, and particularly preferably > 86%.

In the present invention, the polyester may be prepared by a conventional technical method in the art. Exemplarily, the method includes the following steps:
allowing the component A and the component B to undergo an esterification reaction at a temperature of 140-220°C and a pressure of 0.5-2 bar for 1-6 h to obtain an esterification product; allowing the esterification product to undergo a pre-polycondensation reaction at a temperature of 220-270°C and a pressure of 0.1-1 bar for 40-120 min to obtain a pre-polycondensation product; and then allowing the pre-polycondensation product to undergo a polycondensation reaction at a temperature of 230-280°C and a pressure of 1-5 mbar for 120-180 min, and performing slicing and drying to obtain the polyester.

In the present invention, raw materials for the esterification reaction further include a cross-linking agent, where the cross-linking agent includes at least one of tartaric acid, citric acid, malic acid, trimethylolpropane, trimethylolethane, pentaerythritol, polyether triol, glycerol, 1,3,5-benzenetricarboxylic acid, 1,2,4-benzenetricarboxylic acid, 1,2,4-benzenetricarboxylic anhydride, 1,2,4,5-benzenetetracarboxylic acid, or pyromellitic dianhydride; and based on a mass of 100 wt% of a finished product of the polyester, a mass percentage content of the cross-linking agent is 0.05-1 wt%.

In the present invention, the pre-polycondensation reaction further includes a catalyst; the catalyst may be a tin compound, an antimony compound, a cobalt compound, a lead compound, a zinc compound, an aluminum compound, or a titanium compound, more preferably the zinc compound, the aluminum compound, or the titanium compound, and most preferably the titanium compound; the titanium compound may be tetrabutyl titanate or tetraisopropyl titanate; and a total mass of the catalyst is 0.001-1 wt% of a mass of a polycondensation product.

The polyester of the present invention also has biodegradability.

For the present invention, if a substance or a substance mixture shows, as defined in DIN EN 13432, a biodegradation percentage degree of at least 90%, the substance or the substance mixture has the characteristic of "biodegradability".

Biodegradation usually leads to decomposition of a polyester or a polyester mixture within a reasonable inspection period. Degradation may occur through an enzymatic, hydrolytic, or oxidative pathway, and/or through exposure to electromagnetic radiation such as ultraviolet radiation, and is most commonly caused by exposure to microorganisms such as bacteria, yeast, fungi, or algae. By mixing the polyester with compost and storing the same for a specific period of time, the biodegradability may be quantified. For example, according to DIN EN 13432:2000, during composting, air free of CO₂ is introduced into mature compost, and the compost undergoes a specific temperature process. Herein, the biodegradability is defined as a biodegradation percentage degree represented by a ratio of a net amount of CO₂ released by a sample (after subtracting an amount of CO₂ released by compost without the sample) to a maximum amount of CO₂ that can be released by the sample (calculated based on a carbon content in the sample).

Additionally, other methods for determining the biodegradability are described in ASTM D5338:2021 and ASTM D6400:1999.

The numerical ranges in the present invention include not only the above-mentioned enumerated point values but also any point values within the above-mentioned numerical ranges that are not enumerated, and due to space limitations and for the sake of simplicity, all specific point values included within the ranges are not exhaustively enumerated in the present invention.

Compared with the prior art, the present invention has the following beneficial effects.

The bio-based succinic acid composition provided by the present invention contains a specific content of the metal ion, so that the polyester prepared and obtained by using the bio-based succinic acid composition as a raw material has better mechanical properties and thermal stability, still has a higher strength retention rate after treatment at high temperature for a period of time, and has a low cost.

### DETAILED DESCRIPTION OF EMBODIMENTS

The technical solutions of the present invention are further described below through specific embodiments. Those skilled in the art should understand that examples described are merely to facilitate an understanding of the present invention and should not be regarded as specific limitations to the present invention.

Unless otherwise specified, raw materials used in the present invention are as follows:
1,4-butanediol: purchased from Xinjiang Markorchem Co., Ltd., with a purity of 99.7%;
glycerol: purchased from Aladdin;
adipic acid: purchased from Chongqing Huafeng Chemical Co., Ltd.;
tetrabutyl titanate: purchased from Jianyi Chemical Import and Export Co., Ltd.; and
732-type cation exchange resin: purchased from Tianjin Xijinna Environmental Protection Material Technology Co., Ltd.

In the present invention, in a preparation method, the concentration of succinic acid in an obtained succinic acid stock solution, an aqueous solution of succinic acid, and a succinic acid filtrate is tested by liquid chromatography, and a specific test method refers to a method disclosed in CN118459331A.

### Example 1

This example provides a bio-based succinic acid composition, which includes bio-based succinic acid and a metal ion. The content of the bio-based succinic acid and the composition and content of the metal ion are shown in Table 1, and a remainder in the composition includes water and other impurity acids remaining in the process of preparing the succinic acid composition.

This example provides a method for preparing the bio-based succinic acid composition, which specifically includes the following steps:
(1) using a biomass resource as a raw material to prepare and obtain a succinic acid stock solution with a succinic acid content of 78 g/L, where a specific preparation method may adopt a conventional method in the art, and for example, may refer to a method in CN118459331A for preparation;
(2) filtering the succinic acid stock solution obtained in the step (1), and based on a volume of 1 L of the succinic acid stock solution, adding 32 g of diisooctyl phosphate for 5-stage forward cross-current extraction to obtain a succinic acid-loaded organic phase; and then, based on a volume of 1 L of the succinic acid-loaded organic phase, subjecting the succinic acid-loaded organic phase to 5-stage reverse cross-current extraction with 65 g of deionized water to obtain an aqueous solution of succinic acid;
(3) allowing the aqueous solution of succinic acid obtained in the step (2) to pass through a cation exchange resin column at a flow rate of 1 BV/h, and performing elution with water with a mass 6 times that of the aqueous solution of succinic acid to obtain a succinic acid effluent;
(4) subjecting the succinic acid effluent obtained in the step (3) to decolorization with activated carbon at 75°C for 60 min, where based on a volume of 1 L of the succinic acid effluent, a mass of the activated carbon is 8 g (that is, the mass of the activated carbon is 8 g/L); and then, performing ultrafiltration at 50°C using an ultrafiltration membrane with a pore size of 40 nm to obtain a succinic acid filtrate; and
(5) subjecting the succinic acid filtrate obtained in the step (4) to reduced-pressure distillation treatment at a distillation temperature of 70°C and a pressure of -0.07 - -0.1 MPa, stopping the distillation when the content of succinic acid is greater than 15 wt%, and subjecting the concentrated succinic acid solution to cooling crystallization at a low temperature (4°C) to obtain a white succinic acid crystal, thereby obtaining the bio-based succinic acid composition.

### Examples 2-6 and Comparative Examples 1-6

Examples 2-6 and Comparative Examples 1-6 respectively provide a bio-based succinic acid composition, with the composition and process parameters shown in Table 1 and Table 2, where in a preparation method, a step (1) and a step (5) are the same as those in Example 1. In Table 1 and Table 2, a numerical value corresponding to the type of a forward extractant in a step (2) is a mass of the forward extractant (unit: g) based on a volume of 1 L of a succinic acid stock solution; a numerical value corresponding to deionized water is a mass of the deionized water (unit: g) based on a volume of 1 L of a succinic acid-loaded organic phase; and in a step (3), a use amount of water for elution is a multiple of a mass of an aqueous solution of succinic acid.

In Table 1 and Table 2, "/" represents that the component is absent or the step is not performed.

### Example 7

This example provides a bio-based succinic acid composition, with the composition shown in Table 1. The only difference in the preparation method from Example 1 is that the succinic acid stock solution is an equal volume of an aqueous solution of the bio-based succinic acid composition provided in Comparative Example 1. Other step parameters are all the same as those in Example 1.

In the present invention, the pH value of the bio-based succinic acid composition is tested by adopting the following method: weighing and adding 1.1800±0.0020 g of the bio-based succinic acid composition into 80 mL of deionized water, stirring the composition until the composition is completely dissolved, transferring a resulting mixture into a 100 mL volumetric flask, adding deionized water to reach a calibration line of the volumetric flask to obtain a 0.1 mol/L bio-based succinic acid composition solution, and testing the pH value of the bio-based succinic acid composition solution in parallel twice using a pH meter to obtain a mean value and retain three decimal places.

The content of a metal cation in the succinic acid composition is tested according to the following procedure by referring to US EPA Method 3052:1996 and adopting ICP-OES analysis: weighing approximately 0.1 g of the bio-based succinic acid composition, adding 5 mL of nitric acid to completely immerse the bio-based succinic acid composition, then dropping 1.0 mL of hydrogen peroxide to carry out a reaction for 2 min, sealing a resulting mixture in a microwave digestion tank for digestion at 210°C for 3 h, cooling to room temperature, performing filtration with a 0.45 µm filter membrane, diluting the mixture to 50 mL with distilled water, and testing the mixture by ICP-OES.

In the present invention, the mass of bio-based succinic acid in the bio-based succinic acid composition is tested by referring to a method for determining the content of succinic acid in Part 4.3 of GB/T 34686-2017.

**Table 1**

| | | | Exa mple 1 | Exa mple 2 | Exa mple 3 | Exa mple 4 | Exa mple 5 | Exa mple 6 | Exa mple 7 | Compa rative Exampl e 1 |
|---|---|---|---|---|---|---|---|---|---|---|
| Mass of bio-based succinic acid (%) | | | 99.8 8 | 99.8 3 | 99.8 0 | 99.6 5 | 99.9 2 | 99.9 3 | 99.7 9 | 99.52 |
| Mass of a metal ion (ppm) | | Na⁺ | 17.9 | 24.6 | 33.9 | 51.2 | 8.1 | 4.4 | 34.4 | 128.2 |
| | | K⁺ | 11.3 | 15.2 | 24.6 | 35.1 | 10.2 | 6.6 | 25.3 | 130.5 |
| | | Mg²⁺ | 21.9 | 25.3 | 33.3 | 42.6 | 12.8 | 6.2 | 37.1 | 137.3 |
| | | Ca²⁺ | 102. 1 | 121. 0 | 160. 9 | 213. 7 | 72.9 | 29.9 | 163. 4 | 552.1 |
| Total mass of metal ions (ppm) | | | 153. 2 | 186. 1 | 252. 7 | 342. 6 | 104. 0 | 47.1 | 260. 0 | 687.1 |
| pH value | | | 2.71 2 | 2.43 1 | 2.68 6 | 2.67 7 | 2.32 2 | 2.73 1 | 2.69 1 | 2.693 |
| St ep (2 ) | Forward extracta nt | Diisoo ctyl phosph ate | 32 g | 27 g | 22 g | 22 g | 36 g | 50 g | 32 g | 32 g |
| | | Numbe r of times of extracti on | 5 | 5 | 3 | 2 | 5 | 8 | 5 | 5 |
| | Reverse extracta nt | Deioni zed water | 65 g | 53 g | 45 g | 35 g | 65 g | 85 g | 65 g | 65 g |
| St ep (3 ) | Flow rate of a cation exchange resin column (BV/h) | | 1 | 1.4 | 2.2 | 2.7 | 1 | 1 | 1 | / |
| | Elution | Use amount of water | 6 times | 4 times | 3 times | 3 times | 5 times | 6 times | 6 times | / |
| St ep (4 ) | Decolori zation with activated carbon | Temper ature (°C) | 75 | 70 | 70 | 70 | 70 | 75 | 75 | 75 |
| | | Time (min) | 60 | 45 | 45 | 60 | 60 | 60 | 60 | 60 |
| | | Use amount (g/L) | 8 | 4 | 2.5 | 7 | 7 | 8 | 8 | 8 |
| | Filtratio n with an ultrafiltr ation membra ne | Temper ature (°C) | 50 | 50 | 45 | 50 | 50 | 50 | 50 | 50 |
| | | Pore size (nm) | 40 | 40 | 30 | 40 | 40 | 40 | 40 | 40 |

**Table 2**

| | | | | Compar ative Example 2 | Compar ative Example 3 | Compar ative Example 4 | Compar ative Example 5 | Compar ative Example 6 |
|---|---|---|---|---|---|---|---|---|
| Mass of bio-based succinic acid (%) | | | | 99.13 | 99.34 | 99.28 | 99.55 | 99.30 |
| Mass of a metal ion (ppm) | | Na⁺ | | 80.5 | 70.7 | 74.8 | 63.2 | 78.3 |
| | | K⁺ | | 94.1 | 84.1 | 88.5 | 76.1 | 90.2 |
| | | Mg²⁺ | | 103.4 | 83.4 | 94.7 | 78.6 | 86.4 |
| | | Ca²⁺ | | 272.3 | 243.2 | 260.2 | 232.7 | 251.5 |
| Total mass of metal ions (ppm) | | | | 550.3 | 481.4 | 518.2 | 450.6 | 506.4 |
| pH value | | | | 2.756 | 2.743 | 2.748 | 2.730 | 2.751 |
| Ste p (2) | Forward extractant | | Diisooctyl phosphate | / | 15 g | / | 32 g | 32 g |
| | | | Trioctylpho sphine oxide | / | / | 32 g | / | / |
| | | | Number of times of extraction | / | 5 | 5 | 5 | 5 |
| | Reverse extractant | | Deionized water | / | 20 g | 65 g | 65 g | 65 g |
| Ste p (3) | Flow rate of a cation exchange resin column (BV/h) | | | 1 | 1 | 1 | 3.5 | 1 |
| | Elution | | Use amount of water | 6 times | 6 times | 6 times | 6 times | 1 time |
| Ste p (4) | Decoloriz ation with activated carbon | | Temperatur e (°C) | 75 | 75 | 75 | 75 | 75 |
| | | | Time (min) | 60 | 60 | 60 | 60 | 60 |
| | | | Use amount (g/L) | 8 | 8 | 8 | 8 | 8 |
| | Filtration with an ultrafiltrat ion membrane | | Temperature (°C) | 50 | 50 | 50 | 50 | 50 |
| | | | Pore size (nm) | 40 | 40 | 40 | 40 | 40 |

ND represents that the content of a relevant element is not detected.

### Application Example 1

This application example provides a polyester, where raw materials for preparing the polyester include 350 kg of 1,4-butanediol, 327 kg of succinic acid, 1.8 kg of glycerol, and 0.38 kg of tetrabutyl titanate; and the succinic acid is the bio-based succinic acid composition provided in Example 1.

A method for preparing the polyester includes the following steps:
S1: physically mixing the succinic acid, the 1,4-butanediol, and the glycerol, and after the mixing is completed, transferring a resulting mixture into an esterification reactor to carry out an esterification reaction at a temperature of 170°C and a pressure of 1.0 bar for 4 h to obtain an esterification product;
S2: transferring the esterification product obtained in the step S1 into a vertical reactor with a stirrer, adding 0.38 kg of the tetrabutyl titanate, and allowing the reaction mixture to undergo a pre-polycondensation reaction at 250°C and at an internal reactor pressure of 0.3 bar for 70 min to obtain a pre-polycondensation product; and
S3: transferring the pre-polycondensation obtained in the step S2 into a horizontal reactor with a stirrer to carry out a polycondensation reaction at a temperature of 253°C and a pressure of 2.5 mbar for 160 min, and performing slicing and drying to obtain the polyester.

### Application Examples 2-7

Application Examples 2-7 respectively provide a polyester, where the only difference from Application Example 1 is that the succinic acid is the bio-based succinic acid composition provided in Examples 2-7, respectively. Other raw materials, use amounts, and preparation methods are all the same as those in Application Example 1.

### Application Example 8

Application Example 8 provides a polyester, where the differences from Application Example 1 are that with a total molar amount of dicarboxylic acids remaining unchanged, the polyester includes 75 mol% of succinic acid and 25 mol% of adipic acid, and raw materials for preparing the polyester further include 350 kg of 1,4-butanediol, 2.8 kg of glycerol, and 0.52 kg of tetrabutyl titanate. A method for preparing the polyester includes the following steps:
S1: physically mixing the succinic acid, the adipic acid, the 1,4-butanediol, and the glycerol, and after the mixing is completed, transferring a resulting mixture into an esterification reactor to carry out an esterification reaction at a temperature of 200°C and a pressure of 1.5 bar for 3 h to obtain an esterification product;
S2: transferring the esterification product obtained in the step S1 into a vertical reactor with a stirrer, adding the tetrabutyl titanate, and allowing the reaction mixture to undergo a pre-polycondensation reaction at 240°C and at an internal reactor pressure of 0.5 bar for 100 min to obtain a pre-polycondensation product; and
S3: transferring the pre-polycondensation obtained in the step S2 into a horizontal reactor with a stirrer to carry out a polycondensation reaction at a temperature of 248°C and a pressure of 4 mbar for 130 min, and performing slicing and drying to obtain the polyester.

### Application Example 9

Application Example 9 provides a polyester, where the differences from Application Example 1 are that with a total molar amount of dicarboxylic acids remaining unchanged, the polyester includes 66 mol% of succinic acid and 34 mol% of adipic acid, and raw materials for preparing the polyester further include 350 kg of 1,4-butanediol, 3.8 kg of glycerol, and 0.58 kg of tetrabutyl titanate. A method for preparing the polyester includes the following steps:
S1: physically mixing the succinic acid, the adipic acid, the 1,4-butanediol, and the glycerol, and after the mixing is completed, transferring a resulting mixture into an esterification reactor to carry out an esterification reaction at a temperature of 210°C and a pressure of 1.8 bar for 2 h to obtain an esterification product;
S2: transferring the esterification product obtained in the step S1 into a vertical reactor with a stirrer, adding the tetrabutyl titanate, and allowing the reaction mixture to undergo a pre-polycondensation reaction at 260°C and at an internal reactor pressure of 0.8 bar for 80 min to obtain a pre-polycondensation product; and
S3: transferring the pre-polycondensation obtained in the step S2 into a horizontal reactor with a stirrer to carry out a polycondensation reaction at a temperature of 252°C and a pressure of 3.5 mbar for 150 min, and performing slicing and drying to obtain the polyester.

### Comparative Application Examples 1-6

Comparative Application Examples 1-6 provide a polyester, where the only difference from Application Example 1 is that the succinic acid is the bio-based succinic acid composition provided in Comparative Examples 1-6, respectively. Other raw materials, use amounts, and preparation methods are all the same as those in Application Example 1.

### Performance tests

(1) Melt Index: The melt indices of the polyesters provided in Application Examples 1-9 and Comparative Application Examples 1-6 were tested under test conditions of 190°C and 2.16 kg by referring to the standard ISO 1133-2-2012.
(2) The polyesters provided in Application Examples 1-9 and Comparative Application Examples 1-6 were injection-molded into mechanical specimens according to requirements of the standard GB/T 1040-92, where temperatures of first, second, third, and fourth sections of an injection molding machine were 180°C, 180°C, 180°C, and 180°C, respectively. According to the standard ISO 527-2-2012, an initial tensile strength (designated as L₀) of a specimen and a tensile strength (designated as L₁) of the specimen injection-molded into a mechanical specimen after heat retention in a cavity of the injection molding machine at 180°C for 10 min were tested, and a retention rate of tensile strength (ΔL) was calculated as ΔL=L₁/L₀×100%, where conditions for testing the tensile strength included an ambient temperature of 23±2°C and a tensile rate of 50 mm/min.

Specific test results are shown in Table 3.

**Table 3**

| | Melt index (g/10 min) | Heat retention stability | | |
|---|---|---|---|---|
| | | L₀ (MPa) | L₁ (MPa) | ΔL (%) |
| Application Example 1 | 3.7 | 38.2 | 32.9 | 86.1 |
| Application Example 2 | 4.2 | 37.8 | 31.4 | 83.0 |
| Application Example 3 | 5.2 | 37.6 | 30.5 | 81.1 |
| Application Example 4 | 6.2 | 37.5 | 27.8 | 74.1 |
| Application Example 5 | 3.3 | 38.8 | 34.2 | 88.1 |
| Application Example 6 | 6.9 | 37.4 | 26.4 | 70.6 |
| Application Example 7 | 5.4 | 37.6 | 29.7 | 78.1 |
| Application Example 8 | 4.2 | 25.4 | 21.3 | 83.8 |
| Application Example 9 | 4.0 | 22.1 | 18.6 | 84.3 |
| Comparative Application Example 1 | 10.6 | 35.7 | 22.3 | 62.5 |
| Comparative Application Example 2 | 8.8 | 36.0 | 24.1 | 66.9 |
| Comparative Application Example 3 | 7.7 | 36.7 | 25.0 | 68.1 |
| Comparative Application Example 4 | 8.3 | 36.1 | 24.5 | 67.9 |
| Comparative Application Example 5 | 7.4 | 36.9 | 25.3 | 68.6 |
| Comparative Application Example 6 | 8.2 | 36.3 | 24.7 | 68.0 |

It can be seen from Table 3 that the polyester prepared and obtained by using the bio-based succinic acid composition containing a specific content of the metal ion provided by the present invention as a raw material has excellent mechanical properties and heat retention stability, and meanwhile, has a lower melt index. The polyester has a melt index of ≤ 8.0 g/10 min, an initial tensile strength of ≥ 22.1 MPa, and a retention rate of tensile strength of ≥ 70.2% after the heat retention at 180°C for 10 min.

It can be seen from the comparative application examples that the polyester obtained without using the bio-based succinic acid composition provided by the present invention as a raw material has inferior mechanical properties and heat retention stability to those in Application Examples 1-7.

The specific examples described above are to further describe the purposes, technical solutions, and beneficial effects of the present invention in detail. It should be understood that the above descriptions are only specific examples of the present invention and are not intended to limit the present invention, and any modifications, equivalent substitutions, improvements, and the like made within the spirit and principles of the present invention should all be included within the scope of protection of the present invention.

## Claims

1. A bio-based succinic acid composition, comprising bio-based succinic acid and a metal ion, wherein
a mass content of the metal ion in the bio-based succinic acid composition is 10-350 ppm.

2. The bio-based succinic acid composition according to claim 1, wherein the mass content of the metal ion in the bio-based succinic acid composition is 20-280 ppm; and
the metal ion comprises any one or a combination of at least two of Na⁺, K⁺, Mg²⁺, and Ca²⁺.

3. The bio-based succinic acid composition according to claim 1, wherein the metal ion comprises Na⁺, K⁺, Mg²⁺, and Ca²⁺;
a mass content of the Na⁺ or K⁺ in the bio-based succinic acid composition is ≤ 60 ppm;
a mass content of the Mg²⁺ in the bio-based succinic acid composition is ≤ 50 ppm;
a mass content of the Ca²⁺ in the bio-based succinic acid composition is ≤ 220 ppm; and
under conditions of a temperature of 25°C and a concentration of 0.1 mol/L, a pH value of an aqueous solution of the bio-based succinic acid composition is ≤ 2.8.

4. A method for preparing the bio-based succinic acid composition according to any one of claims 1-3, comprising the following steps:
(1) using a biomass resource as a raw material to prepare and obtain a succinic acid stock solution; and
(2) purifying the succinic acid stock solution obtained in the step (1) to obtain the bio-based succinic acid composition, wherein
a method for the purifying comprises an extraction method and an adsorption method using a cation exchange resin column.

5. The preparation method according to claim 4, wherein a content of succinic acid in the succinic acid stock solution is 70-95 g/L;
the extraction method comprises forward cross-current extraction and reverse cross-current extraction;
an extractant for the forward cross-current extraction comprises a phosphate ester extractant;
the phosphate ester extractant comprises at least one of diisooctyl phosphate, diethyl phosphate, or triethyl phosphate;
based on a volume of 1 L of the succinic acid stock solution, a mass of the extractant for the forward cross-current extraction is 20-55 g;
an extractant for the reverse cross-current extraction is water;
based on a volume of 1 L of a solution containing succinic acid obtained by the forward cross-current extraction, a mass of the extractant for the reverse cross-current extraction is 30-95 g;
a number of times of the forward cross-current extraction and the reverse cross-current extraction is each independently ≥ 1 time;
the cation exchange resin column is capable of adsorbing at least one of Na⁺, K⁺, Mg²⁺, and Ca²⁺;
a column loading flow rate of the adsorption method using the cation exchange resin column is 0.5-2.8 BV/h;
after passing through the cation exchange resin column, the adsorption method using the cation exchange resin column further comprises a step of elution with water; and
a mass of the water is 2-7 times that of a solution to be eluted.

6. The preparation method according to claim 4, wherein the biomass resource comprises a plant resource and/or an animal resource;
a method for preparing and obtaining the succinic acid stock solution in the step (1) comprises a microbial fermentation method and/or a chemical conversion method;
the method for the purifying further comprises decolorization with activated carbon and/or filtration;
in the decolorization with activated carbon, based on a volume of 1 L of a solution to be decolorized, a mass of the activated carbon is 0.5-10 g;
a temperature of the decolorization with activated carbon is 60-85°C, and a time is 30-65 min; and
after the purifying, the preparation method further comprises a step of reduced-pressure distillation and/or cooling crystallization.

7. The preparation method according to claim 4, comprising the following steps:
(1) using the biomass resource as the raw material to prepare and obtain the succinic acid stock solution with a succinic acid content of 70-95 g/L;
(2) subjecting the succinic acid stock solution obtained in the step (1) to forward cross-current extraction with a phosphate ester extractant to obtain a succinic acid-loaded organic phase; and then, subjecting the succinic acid-loaded organic phase to reverse cross-current extraction with water to obtain an aqueous solution of succinic acid, wherein based on a volume of 1 L of the succinic acid stock solution, a mass of the phosphate ester extractant is 20-55 g; based on a volume of 1 L of the succinic acid-loaded organic phase, a mass of the water is 30-95 g; and a number of times of the forward cross-current extraction and the reverse cross-current extraction is each independently ≥ 1 time;
(3) allowing the aqueous solution of succinic acid obtained in the step (2) to pass through a cation exchange resin column at a flow rate of 0.5-2.8 BV/h, and performing elution with water with a mass 2-7 times that of the aqueous solution of succinic acid to obtain a succinic acid effluent;
(4) sequentially subjecting the succinic acid effluent obtained in the step (3) to decolorization with activated carbon and filtration to obtain a succinic acid filtrate, wherein based on a volume of 1 L of the succinic acid effluent, a mass of the activated carbon is 0.5-10 g; and
(5) subjecting the succinic acid filtrate obtained in the step (4) to reduced-pressure distillation and cooling crystallization to obtain the bio-based succinic acid composition.

8. A polyester, wherein raw materials for preparing the polyester comprise the bio-based succinic acid composition according to any one of claims 1-3.

9. The polyester according to claim 8, comprising the following components:
component A:
based on a total molar amount of 100 mol% of the component A, being a dicarboxylic acid compound comprising the following components:
al, being 65-100 mol% of bio-based succinic acid and/or a derivative of an ester thereof, and
a2, being 0-35 mol% of adipic acid and/or a derivative of an ester thereof;
and
component B: being 1,4-butanediol with a molar amount at least equal to that of the component A.

10. The polyester according to claim 8, wherein after heat retention at 180°C for 10 min, a retention rate of tensile strength of the polyester is > 70%.
